# EUROPEAN PATENT SPECIFICATION

(11) **EP 3 934 703 B1**
(45) Date of publication and mention of the grant of the patent: **02.09.2026**
(21) Application number: 20819702.0
(22) Date of filing: 02.12.2020
(51) Int. Cl.: A61L 9/03, A61L 9/12, B01D 11/02, B27K 5/00, B27K 5/04, B27K 5/06, A01M 29/00, C11C 5/00, F23D 3/00

(54) **METHOD OF DELIVERING A LIQUID COMPOSITION WITH A FRAGRANCE DISPENSING DEVICE**
VERFAHREN ZUR ABGABE EINER FLÜSSIGEN ZUSAMMENSETZUNG MITTELS EINER DUFTABGABEVORRICHTUNG
PROCÉDÉ DE DISTRIBUTION D'UNE COMPOSITION LIQUIDE AU MOYEN D'UN DISPOSITIF DE DIFFUSION DE PARFUM

(30) Priority: 04.12.2019 US 201962943325 P
(43) Date of publication of application: 12.01.2022
(73) Proprietor: Firmenich SA, 1242 Satigny (CH)
(72) Inventor: DUNBAR, Jermaine, Plainsboro, New Jersey 08536 (US); ALLISON, Gerald, Plainsboro, New Jersey 08536 (US); O'LEARY, Nicholas, Plainsboro, New Jersey 08536 (US)
(74) Representative: Strych, Sebastian
(86) International application number: PCT/EP2020/084320
(87) International publication number: WO 2021/110770

(56) References cited:
- WO-A1-2011/120073
- WO-A1-2018/191181
- WO-A2-2005/076770
- KR-B1- 102 011 504
- US-A1- 2010 147 969
- US-A1- 2013 216 428
- ZHANG YANG ET AL: "INFLUENCE OF HEMICELLULOSE EXTRACTION ON WATER UPTAKE BEHAVIOR OF WOOD STRANDS", 1 October 2010 (2010-10-01), XP055773071, Retrieved from the Internet <URL:https://www.researchgate.net/publication/286958762_Influence_of_hemicellulose_extraction_on_water_uptake_behavior_of_wood_strands> [retrieved on 20210208]

## Description

### FIELD OF THE INVENTION

The present disclosure relates to the field of perfumery. In particular, the present disclosure provides fragrance dispensing emanating substrate and related methods for dispensing fragrance oil or liquid fragrance material into an ambient environment.

### BACKGROUND

Typical emanating substrates that rely on capillary action, such as, for example, reed diffusers, deliver fragrance oil or liquid fragrance material to an ambient environment via diffusion. For example, a lower portion of an emanating substrate may be submerged in a reservoir of fragrance oil and the upper portion of the emanating substrate exposed to the ambient environment. The fragrance oil may be absorbed into the lower portion of the emanating substrate and travels to the upper portion through capillary action. In this manner the entire length of the emanating substrate is saturated with fragrance oil. Once the emanating substrate is saturated, and to some extent during the saturation process, the fragrance oil desorbs from the emanating substrate and diffuses into the ambient environment, delivering the fragrance.

The emanating substrates may comprise natural materials, such as, for example, wood, rattan, willow, or similar plants which, when dried, are capable of absorbing, wicking, and diffusing fragrance oils or liquid fragrance material. Limitations of natural materials include the wicking rate (the rate at which the fragrance oil or liquid fragrance material is absorbed into the lower portion of the emanating substrate and travels to the upper portion through capillary action). Other limitations include the rate of diffusion of the fragrance oil or liquid fragrance material into the ambient environment. This parameter is controlled by the surface area to volume ratio of the emanating substrate. Emanating substrates comprising natural materials typically have low surface area to volume ratios. Additional limitations include a lack of flexibility of the emanating substrate. Other limitations include clogging of the emanating substrate.

WO 2011/120073 A1 discloses a spill-resistant fragrance diffuser. WO 2005/076770 A2 discloses a candle having a body of a meltable fuel and a planar wick. US 2010/147969 A1 discloses a wick fragrance diffuser. Zhang Yang et al. discloses the influence of Hemicellulose Extraction on Water Uptake Behavior of Wood Strands. WO 2018/191181 A1 discloses strong and tough structural wood materials, and methods for fabricating and use thereof.

Therefore, there remains a need for a emanating substrate comprising a natural material having improved wicking and/or diffusion rates for the fragrance oils or liquid fragrance materials.

### SUMMARY

The present invention relates to a method for delivering a liquid composition to an ambient environment comprising providing a fragrance dispensing device, wherein providing the fragrance dispensing device comprises: providing a reservoir having an open end, wherein the reservoir contains a quantity of a liquid composition; and providing an emanating substrate consisting of cellulosic material, wherein providing the emanating substrate comprises: selecting wood as the cellulosic material; chemically treating the wood to remove at least 30, or 40 or 50 % of at least one component selected from the group consisting of lignin and hemicellulose; mechanically hot-pressing the chemically treated cellulosic material so as to increase the density of the chemically treated cellulosic material three-fold; and positioning a first end of the emanating substrate in contact with the open end of the reservoir and a second end in contact with the liquid composition.

### BRIEF DESCRIPTION OF THE FIGURES

While the specification concludes with claims particularly pointing out and distinctly claiming the invention, it is believed that the invention will be better understood from the following description of the accompanying figures wherein:

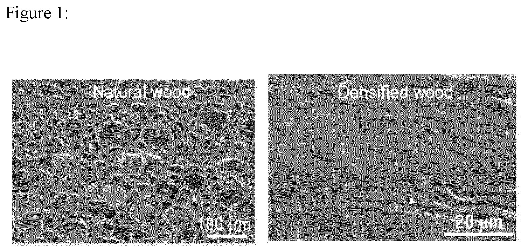

Figure 1 shows a scanning electron micrograph of wood cellulosic material (LEFT) and chemically treated and mechanically hot-pressed wood cellulosic material (RIGHT).

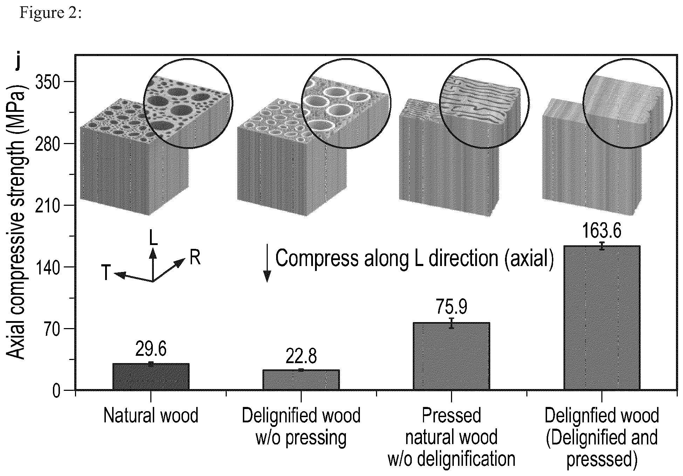

Figure 2 shows the axial compressive strength of wood cellulosic material (FAR LEFT), chemically treated wood cellulosic material (SECOND FROM LEFT), mechanically hot-pressed wood cellulosic material (SECOND FROM RIGHT), and chemically treated and mechanically hot-pressed wood cellulosic material (FAR RIGHT).

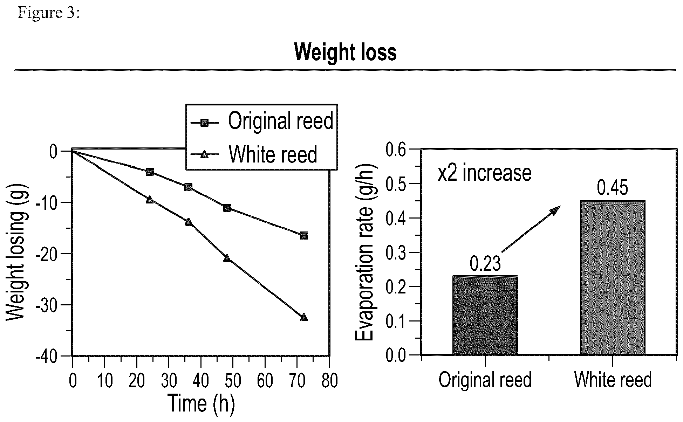

Figure 3 (LEFT) shows the weight loss of a liquid fragrance composition from a fragrance delivery device having an emanating substrate comprising wood (squares) and the weight loss of a liquid fragrance composition from a fragrance delivery device having an emanating substrate comprising chemically treated and mechanically hot-pressed wood cellulosic material (triangles). Figure 3 (RIGHT) shows the rate of evaporation of a liquid fragrance composition from a fragrance delivery device having an emanating substrate comprising wood (left bar) and the rate of evaporation of a liquid fragrance composition from a fragrance delivery device having an emanating substrate comprising chemically treated and mechanically hot-pressed wood cellulosic material (right bar).

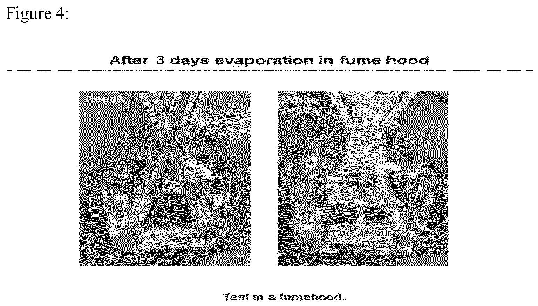

Figure 4 shows the amount of liquid fragrance composition remaining in a fragrance delivery device having an emanating substrate comprising wood after 3 days (LEFT) and the amount of liquid fragrance composition remaining in a fragrance delivery device having an emanating substrate comprising chemically treated and mechanically hot-pressed wood cellulosic material (RIGHT).

### DETAILED DESCRIPTION

In the following description, reference is made to specific embodiments which may be practiced, which is shown by way of illustration. These embodiments are described in detail to enable those skilled in the art to practice the invention described herein, and it is to be understood that other embodiments may be utilized and that logical changes may be made without departing from the scope of the aspects presented herein. The following description of example embodiments is, therefore, not to be taken in a limited sense, and the scope of the various aspects presented herein is defined by the appended claims.

Cellulosic materials that have been treated according to the methods disclosed herein, when used as emanating substrates, provide improved wicking and/or diffusion rates, and may possess improved structural properties and may also be less susceptible to clogging, compared to conventional, non-treated cellulosic materials. Without intending to be limited to any particular theory, treatment of cellulosic materials according to the methods disclosed herein increase the flow of the liquid composition through the emanating substrate, resulting in an improvement of the wicking and/or diffusion rates, thereby increasing the delivery of the liquid composition the an ambient environment. In some aspects, the treatment of cellulosic materials according to the methods disclosed herein reduce the rigidity of the emanating substrate.

As used herein, the term "emanating substrate" refers to a porous substrate configured to deliver fragrance oil or liquid fragrance material to an ambient environment via diffusion.

Referring to Figures 1 to 4, in some aspects, the emanating substrate comprises a cellulosic material that is chemically treated to remove at least one component selected from the group consisting of lignin and hemicellulose from the cellulosic material. Without intending to be limited to any particular theory, removal of the at least one component selected from the group consisting of lignin and hemicellulose creates nanopores between the cellulose nanofibers of the cellulosic material, thereby creating a lumen structure, resulting in an improvement of the wicking and/or diffusion rates, thereby increasing the delivery of the liquid composition the an ambient environment.

The least one component selected from the group consisting of lignin and hemicellulose may be removed from the cellulosic material by immersing the cellulosic material in a boiling alkali solution for a time sufficient to remove the least one component selected from the group consisting of lignin and hemicellulose, followed by at least one wash step to remove the alkali solution.

In some aspects, the alkali solution comprises 2.5M NaOH and 0.4M Na₂SO₃. In an alternate aspect, the alkali solution is ammonia.

In some aspects, the time sufficient is 7, or alternatively, 6, or alternatively, 5, or alternatively, 4, or alternatively, 3, or alternatively, 2, or alternatively, 1 hour.

In some aspects, the time sufficient removes 100%, or alternatively, 90%, or alternatively, 80%, or alternatively, 70%, or alternatively, 60%, or alternatively, 50%, or alternatively, 40%, or alternatively, 30%of the least one component selected from the group consisting of lignin and hemicellulose.

In some aspects, the at least one component selected from the group consisting of lignin and hemicellulose is removed in an amount sufficient to increase the flexibility of the emanating substrate.

In some aspects, the at least one component selected from the group consisting of lignin and hemicellulose is removed in an amount sufficient to change the color of the cellulosic material.

In some aspects, the at least one component selected from the group consisting of lignin and hemicellulose is removed in an amount sufficient to increase the porosity of the emanating substrate.

In some aspects, the increase in porosity increases the rate at which the liquid composition is absorbed into the first end of the emanating substrate.

In some aspects, the increase in porosity increases the rate of diffusion of the liquid composition into the ambient environment.

In some aspects, the increase in porosity increases the surface area to volume ratio of the emanating substrate.

In some aspects, the increased surface area to volume ratio of the emanating substrate increases the rate at which the liquid composition is absorbed into the first end of the emanating substrate.

In some aspects, the increased surface area to volume ratio of the emanating substrate increases the rate of diffusion of the liquid composition into the ambient environment.

Examples of methods suitable to remove the at least one component selected from the group consisting of lignin and hemicellulose from the cellulosic material include the methods disclosed in Song et al., Nature 554, p 224-228 (2018).

Additional examples of methods suitable to remove the at least one component selected from the group consisting of lignin and hemicellulose from the cellulosic material include the methods disclosed in Paril et al., European Journal of Wood and Wood Products, 72, pg 583-591 (2014).

The chemically treated cellulosic material is further treated to increase the density of the chemically treated cellulosic material.

The further treatment comprises mechanical hot-pressing.

The further treatment increases the density of the chemically treated cellulosic material 3 fold.

Examples of methods suitable to mechanically hot press the chemically treated cellulosic material include the methods disclosed in Song et al., Nature 554, pg 224-228 (2018).

Additional examples of methods suitable to mechanically hot press the chemically treated cellulosic material include the methods disclosed in Sandberg et al., Wood Material Science and Engineering 8, pg 64-88 (2013).

Additional examples of methods suitable to mechanically hot press the chemically treated cellulosic material include the methods disclosed in Fang et al., European Journal of Wood and Wood Products, 70, pg 155-163 (2012).

Additional examples of methods suitable to mechanically hot press the chemically treated cellulosic material include the methods disclosed in Paril et al., European Journal of Wood and Wood Products, 72, pg 583-591 (2014).

Additional examples of methods suitable to mechanically hot press the chemically treated cellulosic material include the methods disclosed in Navi et al., MRS Bulletin, 29, pg 332-336 (2004).

Additional examples of methods suitable to mechanically hot press the chemically treated cellulosic material include the methods disclosed in Kutnar et al., Wood Science and Technology 46, pg 73-88 (2012).

Referring to Figure 4, one presented herein provides an emanating substrate comprising chemically treated cellulosic material, the emanating substrate having a first end and a second end that contacts a liquid composition;
wherein the chemical treatment removes at least one component selected from the group consisting of lignin and hemicellulose from the cellulosic material.

Referring to Figure 4, the fragrance dispensing device, comprises:
a) a reservoir having an open end, wherein the reservoir contains a quantity of a liquid composition; and
b) an emanating substrate comprising chemically treated cellulosic material, the emanating substrate having a first end that contacts the open end of the reservoir, and a second end in contact with the liquid composition;
   wherein the chemical treatment removes at least one component selected from the group consisting of lignin and hemicellulose from the cellulosic material.

Disclosed is a candle comprising an emanating substrate comprising chemically treated cellulosic material, and a meltable fuel in direct contact with the an emanating substrate comprising chemically treated cellulosic material. In one aspect, the meltable fuel comprises a wax.

As used herein, the term "liquid composition" refers to a liquid composition which is at least partially volatile, i.e. can evaporate, and which is able to impart a benefit to the anbient environment. It may be a substantially non-aqueous liquid comprising odorant materials, or it may also contain a certain amount of water. It may be a molten wax.

In some aspects, the liquid composition contains at least one active ingredient. The ingredient is capable of imparting a benefit to ambient environment, and may be accompanied by optional ingredients which can be beneficial to the active volatile material. In other words the liquid composition contains an active volatile material, comprising at least one ingredient, and optionally one or more ingredients selected from the group consisting of solvents, thickeners, anti-oxidants, dyes, bittering agents and UV inhibitors.

As the active volatile material, there can be used, for example, a perfume. Other suitable active volatile materials can be deodorizing or sanitizing agents or insect repellents or any other active materials capable of imparting perceptible and desirable benefits to the quality of the air into which they are diffused.

In one aspect, the active volatile material is a perfume. As perfume there can be used any ingredient or mixture of ingredients currently used in perfumery, i.e. capable of exercising a perfuming action, meaning modifying or imparting the odor of the surrounding air. This means that a malodor counteracting composition, capable of reducing or suppressing a large variety of malodors, such as body malodor, tobacco malodor, kitchen or bathroom malodor for example, are also understood herein as being comprised in the "perfume", "fragrance" or "perfuming composition" definition. Often, such a perfuming composition will be a more or less complex mixture of ingredients of natural or synthetic origin. The nature and type of the ingredients do not warrant a more detailed description here, which in any case would not be exhaustive, the skilled person being able to select them on the basis of its general knowledge and according to intended use or application and the desired organoleptic effect, deodorizing, perfuming, sanitizing or other. In general terms, these perfuming ingredients belong to chemical classes as varied as alcohols, baldheads, ketenes, esters, ethers, acetates, nitrides, terrene hydrocarbons, nitrogenous or sulfurous heterocyclic compounds and essential oils of natural or synthetic origin. Many of these ingredients are in any case listed in reference texts such as the book by S. Arcade, Perfume and Flavor Chemicals, 1969, Montclair, N.J., USA, or its more recent versions, or in other works of a similar nature, as well as in the abundant patent literature in the field of perfumery and malodor counteraction. Many are known to possess malodor counteracting and/or antibacterial activity so that, in addition to being capable of perfuming, and thus imparting a pleasant smell to, the surrounding air, they also help purify and sanitize the latter, and/or remove any malodor (i.e. unpleasant smell) thereof.

Natural oils such as lavender, cedar, lemon and other essential oils and extracts may also be employed in the devices disclosed herein.

Although special mention has been made hereinabove of the perfuming effect that can be exerted by the devices of the invention, the same principles apply to analogous devices for the diffusion of deodorizing or sanitizing vapors, the perfume being replaced by a deodorizing composition, an antibacterial, an insecticide, an insect repellent or an insect attractant, or a so-called mothproofed device. By the term "sanitizing vapors", we refer here not only to the vapors of those substances which can enhance the degree of acceptance of the air surrounding the observer, but also to those substances which can exert an attractant or repellent effect towards certain species of insects, for instance towards houseflies or mosquitoes, or else, which can have bactericide or bacteriostatic activity. It goes without saying that mixtures of such agents can also be used.

The total amount of active volatile material in the active composition may be comprised between 20% and 100%, alternatively between 30% and 70%, of the weight of the active composition.

As anticipated above, the active composition may also contain some optional ingredients acting as, for example, solvents, thickeners, anti-oxidants, dyes, bittering agents and UV inhibitors.

As non-limiting examples of useful UV-inhibitor ingredients, one can cite benzophenones, diphenylacrylates or cinnamates such as those available under the trade name UVINUL^{®} (origin: BASF AG).

The total amount of UV-inhibitors present in the active composition may vary between 0.0% and 0.5%, alternatively between 0.01% and 0.4%, the percentages being relative to the total weight of the active composition.

The presence of one or more solvents may be useful to have a single-phase liquid and/or to modulate the speed of evaporation of the active material into the ambient environment. The solvents may belong to the families of isoparaffins, paraffins, hydrocarbons, namely glycols, glycol ethers, glycol ether esters, esters or ketones.

Examples of suitable commercially available solvents are known under the tradename ISOPAR^{®} H, J, K, L, M, P or V (isoparaffins; origin: Exxon Chemical), NORPAR^{®} 12 or 15 (paraffins; origin: Exxon Chemical), EXXSOL^{®} D 155/170, D 40, D 180/200, D 60, D 70, D 80, D 100, D 110 or D 120 (de-aromatized hydrocarbons; origin: Exxon Chemical), DOWANOL^{®} PM, DPM, TPM, PnB, DPnB, TPnB, PnP or DPnP (glycol ethers; origin: Dow Chemical Company), EASTMAN^{®} EP, EB, EEH, DM, DE, DP or DB (glycol ethers; origin: Eastman Chemical Company), DOWANOL^{®} PMA or PGDA (glycol ether esters; origin: Dow Chemical Company) or EASTMAN^{®} EB acetate, EASTMAN^{®} DE acetate, EASTMAN^{®} DB acetate, EASTMAN^{®} EEP (all glycol ether esters; all origin: Eastman Chemical Company) or yet 3-methoxy-3-methyl-1-butanol, also known as solvent MMB and available from a variety of suppliers.

Other examples of solvents useful to the invention are dipropylene glycol, propylene glycol, ethylene glycol ethyl ether acetate, ethylene glycol diacetate, isopropyl myristate, diethyl phthalate, 2-ethylhexyl acetate, methyl n-amyl ketone or di-isobutyl ketone.

The total amount of such solvents present in the active composition may vary between 0.0% and 80%, alternatively between 30% and 70%, the percentages being relative to the weight of the active composition. In some aspect, perfuming compositions comprise at least 30% by weight of perfume and not more than 70% by weight of any such solvents.

In some aspects, at least 60% of total weight of the active composition is made of ingredients having a vapor pressure comprised between 4 Pa and 270 Pa, the vapor pressure being measured at 20° C. and a pressure of 760 mmHg. The described requirement in the formulation of the active composition ensures that a relatively constant composition is maintained over the lifetime of the device and that the active composition evaporates at a relatively steady rate during the life of the product.

In some aspects, at least 80% of total weight of the active composition is made of ingredients having a vapor pressure comprised between 4 Pa and 270 Pa.

As non-limiting examples of useful antioxidant ingredients, one can cite the sterically hindered amines, i.e. the derivatives of the 2,2,6,6-tetramethyl-piperidine, such as those known under the tradename UVINUL^{®} (origin: BASF AG) or TINUVIN^{®} (origin: Ciba Specialty Chemicals), as well as the alkylated hydroxyarene derivatives, such as butylated hydroxytoluene (BHT).

The total amount of antioxidants present in the active composition may vary between 0.0% and 10%, alternatively between 1% and 4%, the percentages being relative to the weight of the active composition.

Dyes are other optional ingredients of the active composition. Suitable dyes are oil-soluble and can be found in the Colour Index International, published by The Society of Dyers and Colourist. Non-limiting examples of suitable dyes are derivatives of the anthraquinone, methine, azo, triarylmethane, triphenylmethane, azine, aminoketone, spirooxazine, thioxanthene, phthalocyanine, perylene, benzopyran or perinone families.

Examples of such dyes which are commercially available are known under the tradename SANDOPLAST^{®} Violet RSB, Violet FBL, Green GSB, Blue 2B or SAVINYL^{®} Blue RS (all anthraquinone derivatives; origin: Clariant Huningue S.A.), OILSOL^{®} Blue DB (anthraquinone; origin: Morton International Ltd.), SANDOPLAST^{®} Yellow 3G (methine; origin: Clariant Huningue S.A.), SAVINYL^{®} Scarlet RLS (azo metal complex; origin: Clariant Huningue S.A.), OILSOL^{®} Yellow SEG (monoazo; origin: Morton International Ltd.), FAT ORANGE^{®} R (monoazo; origin: Hoechst AG), FAT RED^{®} 5B (diazo; origin: Hoechst AG), NEOZAPON^{®} Blue 807 (phtalocyanine; origin: BASF AG), FLUOROL^{®} Green Golden (perylene; origin: BASF AG).

The total amount of dyes present in the active composition may vary between 0.0% and 0.5%, alternatively between 0.005% and 0.05%, the percentages being relative to the weight of the active composition.

The presence of a bittering agent may be desirable in order to render the product unpalatable, making it less likely for the active composition to be ingested, especially by young children. One can cite, as non-limiting examples, isopropyl alcohol, methyl ethyl ketone, methyl n-butyl ketone or yet a denatonium salt such as the denatonium benzoate known also under the trademark BITREX^{™} (origin: Mac Farlan Smith Ltd.).

The bittering agent may be incorporated in the active composition in a total amount comprised between 0.0% and 5%, the percentages being relative to the total weight of the active composition. In the case of BITREX^{™} the amount can be comprised between 0.0% and 0.1%, preferably between 10 and 500 ppm of the total weight of the active composition, whereas the other bittering agents above-mentioned are typically used in amounts from 0.5 to 5% by weight, when present.

In some aspects, the liquid composition is a composition selected from the group consisting of: a fragrance material composition, an insect repellant composition, a deodorizing composition, and combinations thereof.

One aspect presented herein provides a method for delivering a liquid composition to an ambient environment comprising providing a fragrance dispensing device according to an aspect presented herein.

The present invention is best illustrated but is not limited to the following examples.

### EXAMPLES

### Example 1: A Fragrance Dispensing Device According to an Aspect Presented Herein

In a first step, a surfactant-water base was prepared based on the formulation described in Table 1 below. All the ingredients were mixed together in a beaker and gently stirred at room temperature. After their complete dissolution, a clear micellar solution was formed.

**Table 1 - Surfactant-water base pre-formulation according to the invention**

| **Ingredient** | **% w/w** |
|---|---|
| Aerosol OT-100 ¹⁾ | 5.60 |
| Glycosperse^{®} O-20 ²⁾ | 2.15 |
| Glycosperse^{®} L-20 ³⁾ | 0.25 |
| Solubilisant LRI ⁴⁾ | 3.34 |
| Deionised Water | 88.66 |
| | 100.00 |

| | |
|---|---|
| ¹⁾ Dioctyl sulfosuccinate sodium salt; origin: Cytec Industries, Inc. ²⁾ Monooleate ethoxylated sorbitol with 20 EO; origin: Lonza Inc. ³⁾ Monolaureate ethoxylated sorbitol with 20 EO; origin: Lonza Inc. ⁴⁾ PPG-26 Buteth-26 & PEG-40 Hydrogenated Castor Oil & Water; origin: LWR Inc. | |

In a second step, an air freshener composition was obtained according to the formula given in Table 2. The above-described surfactant-water base, perfume base and solubilizing-aid ingredient were mixed together in a beaker. This mixture was gently stirred at ambient temperature for a few minutes by means of a magnetic bar stirrer. A translucent liquid crystal phase formed. In the next step, by water dilution, a clear product was instantly formed.

**Table 2 - Liquid formulation according to the invention**

| **Ingredient** | **% w/w** |
|---|---|
| Surfactant-water base | 44.35 |
| Perfume Base ¹⁾ | 6.00 |
| Ajidew^{®} N-50 ²⁾ | 0.35 |
| Sodium Benzoate | 0.10 |
| Deionised Water | 49.20 |
| | 100.00 |

| | |
|---|---|
| ¹⁾ PURE CITRUS 163641; origin: Firmenich SA. ²⁾ Sodium Pyrrolidone Carboxylic Acid 50% aqueous solution; origin: Ajinimoto Inc. | |

Following this procedure a perfume formulation having a surfactant/perfume ratio of 0.84 was obtained.

The perfume formulation was added to a fragrance dispensing device containing either a rattan wick, or a rattan wick that had been chemically treated to remove the at least one component selected from the group consisting of lignin and hemicellulose, and subsequently treated to increase the density of the chemically treated cellulosic material by mechanical hot pressing according to the methods described in Song et al., Nature 554, p 224-228 (2018).

Figure 3 (LEFT) shows the weight loss of the liquid fragrance composition from a fragrance delivery device having a wick comprising rattan (squares) and the weight loss of a liquid fragrance composition from a fragrance delivery devise having a wick comprising chemically treated and mechanically hot-pressed rattan (triangles).

Figure 3 (RIGHT) shows the rate of evaporation of a liquid fragrance composition from a fragrance delivery device having a wick comprising rattan (left bar) and the rate of evaporation of a liquid fragrance composition from a fragrance delivery devise having a wick comprising chemically treated and mechanically hot-pressed rattan (right bar).

Figure 4 shows the amount of liquid fragrance composition remaining in a fragrance delivery device having a wick comprising wood after 3 days (LEFT) and the amount of liquid fragrance composition remaining in a fragrance delivery device having a wick comprising chemically treated and mechanically hot-pressed wood cellulosic material (RIGHT).

Taken together, these data suggest that treatment of cellulosic materials according to the methods disclosed herein increase the flow of the liquid composition through the wick, resulting in an improvement of the wicking and/or diffusion rates, thereby increasing the delivery of the liquid composition the an ambient environment.

## Claims

1. A method for delivering a liquid composition to an ambient environment comprising providing a fragrance dispensing device,
wherein providing the fragrance dispensing device comprises:
a) providing a reservoir having an open end, wherein the reservoir contains a quantity of a liquid composition; and
b) providing an emanating substrate comprising cellulosic material,
wherein providing the emanating substrate comprises:
- selecting wood as the cellulosic material;
- chemically treating the wood to remove at least 30, or 40 or 50 % of at least one component selected from the group consisting of lignin and hemicellulose;
- mechanically hot-pressing the chemically treated cellulosic material so as to increase the density of the chemically treated cellulosic material three-fold; and
- positioning a first end of the emanating substrate in contact with the open end of the reservoir and a second end in contact with the liquid composition.

## Patentansprüche

1. Verfahren zum Abgeben einer flüssigen Zusammensetzung an eine Umgebung, umfassend das Bereitstellen einer Duftstoffabgabevorrichtung,
wobei das Bereitstellen der Duftstoffabgabevorrichtung Folgendes umfasst:
a) Bereitstellen eines Reservoirs mit einem offenen Ende, wobei das Reservoir eine Menge einer flüssigen Zusammensetzung enthält; und
b) Bereitstellen eines Verströmungssubstrats, das Cellulosematerials umfasst,
wobei das Bereitstellen des Verströmungssubstrats Folgendes umfasst:
- Wählen von Holz als Cellulosematerial;
- chemisches Behandeln des Holzes zur Entfernung von mindestens 30 oder 40 oder 50 % mindestens einer Komponente, die aus der Gruppe Lignin und Hemicellulose ausgewählt ist;
- mechanisches Heißpressen des chemisch behandelten Cellulosematerials zur Erhöhung der Dichte des chemisch behandelten Cellulosematerials um das Dreifache; und
- Positionieren eines ersten Endes des Verströmungssubstrats in Kontakt mit dem offenen Ende des Reservoirs und eines zweiten Endes in Kontakt mit der flüssigen Zusammensetzung.

## Revendications

1. Procédé pour distribuer une composition liquide dans un environnement ambiant comprenant la fourniture d'un dispositif de diffusion de parfum,
dans lequel la fourniture du dispositif de diffusion de parfum comprend :
a) la fourniture d'un réservoir ayant une extrémité ouverte, dans lequel le réservoir contient une quantité d'une composition liquide ; et
b) la fourniture d'un substrat à émanation comprenant un matériau cellulosique,
dans lequel la fourniture du substrat à émanation comprend :
- le choix d'un bois comme matériau cellulosique ;
- un traitement chimique du bois pour éliminer au moins 30, ou 40 ou 50 % d'au moins un composant choisi dans le groupe constitué par la lignine et l'hémicellulose ;
- le pressage mécanique à chaud du matériau cellulosique traité chimiquement de façon à multiplier par trois la masse volumique du matériau cellulosique traité chimiquement ; et
- le positionnement d'une première extrémité du substrat à émanation en contact avec l'extrémité ouverte du réservoir et d'une deuxième extrémité en contact avec la composition liquide.
